# EUROPÄISCHE PATENTSCHRIFT

(11) **EP 3 838 243 B1**
(45) Veröffentlichungstag und Bekanntmachung des Hinweises auf die Patenterteilung: **29.01.2025**
(21) Anmeldenummer: 19218866.2
(22) Anmeldetag: 20.12.2019
(51) Int. Cl.: A61F 13/84, A61F 13/53

(54) **INKONTINENZARTIKEL MIT PH-REGULATIONSMITTEL**
INCONTINENCE ARTICLE WITH PH REGULATOR
ARTICLE D'INCONTINENCE DOTÉ D'UN AGENT DE RÉGULATION DU PH

(43) Veröffentlichungstag der Anmeldung: 23.06.2021
(73) Patentinhaber: Paul Hartmann AG, 89522 Heidenheim (DE)
(72) Erfinder: BÄHRLE, Christian, 89542 Herbrechtingen (DE); ESQUERRA, Juan, 08170 Montornès del Vallès (Barcelona) (ES); GAUSE, Enno, 89522 Heidenheim (DE); HIDALGO, Luis, 08170 Montornès del Vallès (Barcelona) (ES); KESSELMEIER, Rüdiger, 89233 Burlafingen (DE); STALTER, Isabel, 89077 Ulm (DE); SWEREV, Maximilian, 86169 Augsburg (DE)
(74) Vertreter: Paul Hartmann AG Patents & Licensing

(56) Entgegenhaltungen:
- WO-A1-2012/121932
- DE-A1- 102007 062 288

## Beschreibung

Die vorliegende Erfindung betrifft einen Inkontinenzartikel für die Aufnahme von Körperausscheidungen mit einem Mittel zur Regulation des pH-Wertes für die bevorzugte Verwendung durch Erwachsene.

Ein häufiges Problem bei fortdauernder Verwendung von Inkontinenzartikeln ist das Auftreten von Hautreizungen oder Hautentzündungen, welche durch den längeren Kontakt der Haut mit Körperausscheidungen wie beispielsweise Urin hervorgerufen werden. Insbesondere bei älteren inkontinenten Personen tritt häufig eine sogenannte Inkontinenzassoziierte Dermatitis (IAD) auf, eine durch Kontakt mit einem Reizstoff wie Urin hervorgerufene Hautentzündung in der perinealen oder perigenitalen Region. Dabei spielen Veränderungen des pH-Wertes der Haut bei Kontakt mit dem alkalischeren Urin eine wesentliche Rolle. Das gehäufte Auftreten von IAD bei älteren Personen korreliert mit einer im Alter geschwächten Hautbarriere und einer verminderten Fähigkeit der Haut, sich zu erneuern. Zudem sind Personen, die aus medizinischen oder Altersgründen unter Inkontinenz leiden, häufig auf die Unterstützung durch Pflegekräfte angewiesen. Besonders in der Nacht steht dabei einer regelmäßigen Überprüfung des verwendeten Inkontinenzartikels zwecks zeitnahen Wechsels nach einem Miktionsereignis das Bedürfnis der betroffenen Person nach einer ungestörten Nachtruhe entgegen. Daher kommt es gerade hier häufig zu einem länger andauernden Kontakt der Haut mit Körperausscheidungen. Bei Personen, bei denen häufige Miktionsereignisse in kurzen Zeitabständen auftreten, wäre es zur Verbesserung der Lebensqualität wie auch aus Kostengründen vorteilhaft, wenn die Inkontinenzartikel nicht zeitnah nach jedem Miktionsereignis gewechselt werden müssten.

Daher besteht insbesondere bei hoher Miktionsfrequenz oder längeren Wechselintervallen ein Bedarf an Inkontinenzartikeln mit verbesserter Hautverträglichkeit.

Inkontinenzartikel für Erwachsene sind seit langem bekannt und weisen häufig ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper auf. Diese können grundsätzlich auf vielfältige Weise ausgestaltet sein, beispielsweise als direkt am Körper anliegende Inkontinenzprodukte oder als saugfähige Inkontinenzunterlagen.

Im Stand der Technik finden sich Ansätze, Inkontinenzartikel mit einem Mittel zur pH-Wert-Kontrolle bereitzustellen. WO2012121932A1 lehrt die Verwendung einer Pufferlösung, die eine schwache Säure oder eine schwache Base und das jeweilige entsprechende Salz umfasst, wie beispielsweise Citronensäure und Natriumcitrat in einem bestimmten Mischungsverhältnis. Der Begriff Natriumcitrat bezeichnet in der allgemeinen Fachsprache das tribasische Natriumsalz der Citronensäure, auch bekannt als Trinatriumcitrat.

Die Erfinder haben erkannt, dass die Verwendung einer Puffermischung aus Citronensäure und Natriumcitrat den Nachteil hat, dass die Herstellung einer homogenen Mischung prozesstechnisch aufwendig ist und es leicht zu zumindest teilweiser Entmischung und ungleichmäßiger Verteilung der einzelnen Komponenten im Produktionsprozess kommen kann. Dadurch kann es zu fehlerhaftem und nicht spezifikationskonformem Eintrag der Komponenten in die üblicherweise in schnelllaufenden Herstellmaschinen produzierten Inkontinenzartikel kommen. Damit ist die Gefahr verbunden, dass die Menge und/oder die Verteilung der Komponenten der Puffermischung - und damit die Sicherstellung der gewünschten Pufferwirkung bei bestimmungsgemäßer Benutzung der Artikel - zwischen einzelnen Inkontinenzartikeln oder innerhalb eines Inkontinenzartikels starken Schwankungen unterlegen ist. Die Gefahr der lokalen Anhäufung von Citronensäure hat zur Folge, dass der pH-Wert des Inkontinenzartikels in diesem Bereich stark sauer reagiert, das heißt dass der pH-Wert in diesem Bereich deutlich unterhalb eines dem Gesundheitszustand der Haut zuträglichen Wertes sinkt.

Der vorliegenden Erfindung liegt daher die Aufgabe zu Grunde, diese Nachteile zu überwinden.

Eine weitere Aufgabe der Erfindung ist es, Inkontinenzartikel mit verbesserter Hautfreundlichkeit bereitzustellen.

Diese Aufgabe wird gelöst durch einen Inkontinenzartikel für die Aufnahme von Körperausscheidungen, umfassend ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper, wobei der Inkontinenzartikel zwischen dem Topsheet und dem Backsheet zumindest bereichsweise ein pH-Regulationsmittel umfasst, wobei das pH-Regulationsmittel Mononatriumcitrat oder Dinatriumcitrat aufweist, wobei das pH-Regulationsmittel partikelförmig ist, wobei mindestens 50 Gewichtsprozent des pH-Regulationsmittels eine Partikelgröße von 10 bis 2000 µm aufweist. Mithin kann das pH-Regulationsmittel vorzugsweise entweder Mononatriumcitrat oder Dinatriumcitrat, oder Mononatriumcitrat und Dinatriumcitrat aufweisen.

Durch den erfindungsgemäßen Inkontinenzartikel kann ein relativ gleichmäßiger pH-Wert über mehrere Miktionsereignisse hinweg erreicht werden. Ein weiterer Vorteil bei der Verwendung von Mononatriumcitrat ist beispielsweise, dass Mononatriumcitrat weniger hygroskopisch ist als beispielsweise Citronensäure und daher kann ein unerwünschter Eintrag von Feuchtigkeit in den Inkontinenzartikel vor der Benutzung vermieden werden. Da Mononatriumcitrat weniger hygroskopisch ist als beispielsweise Citronensäure ergibt sich als weiterer Vorteil eine bessere Rieselfähigkeit, so dass sich Mononatriumcitrat leichter dosieren lässt.

Das Backsheet kann insbesondere von einem atmungsaktiven, jedoch im Wesentlichen flüssigkeitsdichten Folienmaterial gebildet sein. Das Topsheet ist zumindest bereichsweise vorzugsweise von einem auf Vliesbasis beruhenden zumindest bereichsweise flüssigkeitsdurchlässigen Material gebildet.

In einer bevorzugten Ausführungsform weist das pH-Regulationsmittel zusätzlich Trinatriumcitrat auf, mithin kann das pH-Regulationsmittel vorzugsweise entweder Mononatriumcitrat und Trinatriumcitrat, oder Dinatriumcitrat und Trinatriumcitrat, oder Mononatriumcitrat und Dinatriumcitrat und Trinatriumcitrat aufweisen. Dadurch kann eine Feineinstellung der Pufferwirkung eines Inkontinenzartikels je nach angestrebter Benutzungsdauer oder Absorptionskapazität des Artikels erreicht werden, ohne dass wie im Falle der Verwendung von Citronensäure die Gefahr der Entstehung stark sauer reagierender Bereiche besteht.

Vorzugsweise weist das pH-Regulationsmittel solchenfalls eine homogene Mischung der jeweiligen Citrate auf. Das hat den Vorteil, dass lokale Unterschiede in der Pufferwirkung des pH-Regulationsmittels vermieden werden können.

Die Begriffe Mononatriumcitrat, Dinatriumcitrat, und Trinatriumcitrat umfassen sowohl die nicht hydrierte Form als auch Hydrate der jeweiligen Substanz.

In einer bevorzugten Ausführungsform besteht das pH-Regulationsmittel aus Mononatriumcitrat.

In einer weiteren bevorzugten Ausführungsform besteht das pH-Regulationsmittel aus Dinatriumcitrat.

Durch die Verwendung nur einer Komponente als pH-Regulationsmittel ergeben sich prozesstechnische Vorteile, da keine Vermischung verschiedener Komponenten oder Aufrechterhaltung einer homogenen Durchmischung während des Herstellungsprozesses erforderlich ist. Zudem wird eine gleichmäßige Pufferwirkung über die pH-Regulationsmittel enthaltenden Bereiche des Inkontinenzartikels hinweg weiter unterstützt.

Es hat sich als vorteilhaft erwiesen, wenn mindestens 50 Gewichtsprozent des pH-Regulationsmittels eine Partikelgröße von 50 bis 1200 µm, insbesondere von 80 bis 800 µm aufweisen.

Grundsätzlich sind auch pH-Regulationsmittel mit geringerer oder größerer Partikelgröße erhältlich und verwendbar.

Jedoch bieten die beschriebenen Partikelgrößen den Vorteil, dass sich Partikel einer solchen Größe bei Benetzung mit Körperausscheidungen wie einer Miktionsflüssigkeit langsamer auflösen als pH-Regulationsmittel geringerer Partikelgröße, so dass das pH-Regulationsmittel während der Benutzung nicht so schnell ausgewaschen wird und ein pHregulierender Effekt an einer der Haut des Benutzers zugewandten Oberseite des Inkontinenzartikels länger erhalten bleibt.

Zudem lässt sich ein pH-Regulationsmittel der beschriebenen Partikelgröße insbesondere in schnell laufenden Maschinen leichter dosieren und in den Inkontinenzartikel einarbeiten als beispielsweise feinpudrige pH-Regulationsmittel, da es im Herstellungsprozess zu weniger Staubentwicklung und damit verbundenem Materialverlust kommt.

Andererseits bieten Partikel dieser Größe auch den Vorteil, dass sie während der Benutzung des Inkontinenzartikels haptisch weniger wahrnehmbar sind als beispielsweise Granulate mit höherer Partikelgröße, wodurch der Tragekomfort des Inkontinenzartikels verbessert wird.

Alternativ zu partikelförmigem pH-Regulationsmittel kann das pH-Regulationsmittel auch in gelöster Form, insbesondere als wässrige oder alkoholische Lösung in den Inkontinenzartikel eingebracht sein, insbesondere durch Besprühen oder Eintauchen und optional nachfolgendem Trocknen mindestens einer Absorptionskörperschicht.

Als weitere Alternative kann ein zusätzliches und mit dem pH-Regulationsmittel vorbehandeltes, insbesondere flächenhaftes Material, insbesondere Vliesmaterial oder Zellstoffmaterial im Inkontinenzartikel enthalten sein. Vorzugsweise kann ein solches flächenhaftes Material zwischen der Flüssigkeitsaufnahmeschicht und einer Speicherschicht des Absorptionskörpers angeordnet sein.

In einer bevorzugten Ausführungsform beträgt die Menge an pH-Regulationsmittel 10 bis 100 g/m², insbesondere 20 bis 80 g/m², weiter insbesondere 25 bis 60 g/m². Die Menge an ph-Regulationsmittel ist dabei bezogen auf die Fläche des durch das pH-Regulationsmittel überfangenen Bereichs des plan ausgebreiteten Inkontinenzartikels. Die vorstehende Mengenangabe des pH-Regulationsmittels ist so zu verstehen, dass sie sich auf ein wasserfreies pH-Regulationsmittel, beispielsweise nicht hydriertes Mononatriumcitrat, bezieht. Falls das pH-Regulationsmittel ein oder mehrere Hydrate oder wie nachfolgend beschrieben mehr als 5%, insbesondere mehr als 10% additive Substanzen umfasst, wird die Gesamtmenge des pH-Regulationsmittels in g/m² derart angepasst, dass die Gesamtmenge an dem enthaltenen reinen und/oder nicht hydrierten pH-Regulationsmittel der vorstehenden bevorzugten Menge entspricht.

Bevorzugt wird das Mononatriumcitrat oder Dinatriumcitrat oder Trinatriumcitrat im Wesentlichen in reiner Form verwendet, insbesondere mit einem Reinheitsgrad von mindestens 90%, weiter insbesondere mindestens 95%, weiter insbesondere mindestens 98%, weiter insbesondere mindestens 99%, weiter insbesondere 100%. Dadurch können durch Rückstände anderer Substanzen ausgelöste Hautirritationen bei Benutzung des Inkontinenzartikels weitestgehend vermieden werden.

Es ist jedoch auch denkbar, dass das pH-Regulationsmittel eine geringe Menge einer oder mehrerer additiver Substanzen aufweist, beispielsweise Konfektionierungs- und/oder Konditionierungsmittel wie Trennmittel, Stabilisatoren, Staubbindemittel, Antibackmittel, Netzmittel, Koagulationsmittel, Antikoagulationsmittel, adhäsive oder oberflächenaktive Substanzen oder antimikrobielle Substanzen, Farb- oder Duftstoffe oder pH-Indikatoren. Solchenfalls beträgt der Anteil der einen oder mehreren anderen Substanzen an der Gesamtmenge des pH-Regulationsmittels insgesamt bevorzugt weniger als 10%, weiter bevorzugt weniger als 5%, weiter bevorzugt weniger als 2%, weiter bevorzugt weniger als 1%.

Der Absorptionskörper des Inkontinenzartikels ist geeignet und dazu bestimmt, Körperausscheidungen, insbesondere Körperflüssigkeiten, insbesondere Urin aufzunehmen und dauerhaft zu speichern.

Der Absorptionskörper umfasst dazu in vorteilhafter Weise mindestens eine Speicherschicht, die bevorzugt ein superabsorbierendes Polymer (SAP) aufweist.

Alternativ kann der Absorptionskörper auch mehr als eine, insbesondere mindestens zwei Speicherschichten umfassen.

Der Absorptionskörper kann einen oder mehrere Kanäle in einer oder mehr als einer Speicherschicht umfassen.

Der Absorptionskörper kann rechteckig, dreieckig, oval, T-förmig, sanduhrförmig, asymmetrisch oder in einer anderen dem Fachmann als geeignet erscheinenden Form ausgebildet sein.

In einer bevorzugten Ausführungsform enthält der Absorptionskörper superabsorbierendes Polymermaterial (SAP) zu 5 - 100 Gewichtsprozent, vorzugsweise zu 10 - 95 Gewichtsprozent, weiter vorzugsweise zu 15 - 90 Gewichtsprozent, ganz besonders bevorzugt zu 20 - 80 Gewichtsprozent. Typischerweise kann das SAP-Material zumindest das 15-fache, insbesondere das 20-fache seines Gewichts an 0,9 gewichtsprozentiger Kochsalzlösung absorbieren (gemessen nach NWSP 242.0.R2(15)).

Das SAP-Material kann beispielsweise partikelförmig oder faserförmig oder blatt- oder schaumförmig ausgebildet sein.

Die Speicherschicht des Absorptionskörpers kann weitere Materialien, wie Zellstofffasern ("fluff") oder Kunststofffasern enthalten. Denkbar ist weiterhin, die Speicherschicht des Absorptionskörpers durch Anordnung von ein oder mehreren Schichten verschiedenen Materials, insbesondere aus Vliesmaterial auszubilden.

Nach einer bevorzugten Ausführungsform ist die Speicherschicht im Wesentlichen frei von dem pH-Regulationsmittel.

Im Wesentlichen frei von pH-Regulationsmittel bedeutet, dass die jeweilige Schicht des Absorptionskörpers, insbesondere die Speicherschicht, weniger als 10 g/m², insbesondere weniger als 7 g/m², weiter insbesondere weniger als 5 g/m², weiter insbesondere weniger als 2 g/m² pH-Regulationsmittel, weiter insbesondere kein pH-Regulationsmittel enthält. Dadurch wird auf vorteilhafte Weise eine mögliche Funktionsbeeinträchtigung der jeweiligen Schicht, insbesondere der Speicherschicht, insbesondere des in der Speicherschicht bevorzugt enthaltenen SAP-Materials vermieden.

Weiter wird bevorzugt, dass die dem Backsheet unmittelbar zugewandte Schicht des Absorptionskörpers im Wesentlichen frei von dem pH-Regulationsmittel ist. Daraus ergibt sich in vorteilhafter Weise ein höherer Tragekomfort, insbesondere bei einem am Körper anliegenden Inkontinenzartikel, da von der Backsheetseite her ein weicherer haptischer Eindruck des Inkontinenzartikels erreicht wird. Dem Fachmann ist außerdem bekannt, dass partikelförmiges Material abrasive Kräfte ausüben kann, die das im Wesentlichen flüssigkeitsundurchlässige Backsheet, insbesondere eine flüssigkeitsundurchlässige Backsheetfolie des absorbierenden Artikels beschädigen und zu Undichtigkeit des Artikels führen können. Eine im Wesentlichen von pH-Regulationsmittel, insbesondere partikelförmigem pH-Regulationsmittel freie, dem Backsheet unmittelbar zugewandte Schicht hat daher den Vorteil, dass die Gefahr der Beschädigung der Backsheet-Folie im Herstellungsprozess oder während der Benutzung des Inkontinenzartikels verringert wird.

Weiter erweist sich als vorteilhaft, wenn der Inkontinenzartikel eine Flüssigkeitsaufnahmeschicht umfasst, die eine dem Topsheet unmittelbar zugewandte Schicht des Absorptionskörpers bildet. Dadurch kann eine während eines Miktionsereignisses anfallende Flüssigkeitsmenge rasch aufgenommen, verteilt und an die mindestens eine Speicherschicht weitergeleitet werden.

Schichten zur raschen Aufnahme und Verteilung von Körperausscheidungen, sogenannte Flüssigkeitsaufnahme- oder Verteilerschichten sind im Fachgebiet bereits bekannt und bestehen typischerweise aus einem Fasermaterial, insbesondere einem Vliesmaterial. Die Fasern können natürlichen oder künstlichen Ursprungs sein und können von definierter Länge (Stapelfasern) oder kontinuierlich ("endlos") sein oder in-situ gebildet werden. Die Fasern können aus einem einzelnen Polymer oder einer Polymermischung (Einkomponentenfaser) oder aus mehr als einem einzelnen Polymer und/oder einer Polymermischung (Mehrkomponentenfaser) ausgebildet sein.

Eine Mehrkomponentenfaser weist einen Querschnitt auf, der mehr als einen einzelnen Abschnitt umfasst, wobei jeder dieser Abschnitte ein anderes Polymer oder eine andere Polymermischung umfasst. Der Begriff Mehrkomponentenfaser umfasst, ist aber nicht beschränkt auf, eine Zweikomponentenfaser. Die verschiedenen Komponenten von Mehrkomponentenfasern sind in im Wesentlichen unterschiedlichen Bereichen über den Querschnitt der Faser angeordnet und erstrecken sich kontinuierlich über die Länge der Faser. Eine Mehrkomponentenfaser kann einen Gesamtquerschnitt aufweisen, der Teilbereiche der zwei oder mehr unterschiedlichen Komponenten jeglicher Form oder Anordnung aufweist, einschließlich beispielsweise koaxialer Unterabschnitte, Kern- und Hüllenunterabschnitte, nebeneinander liegende Unterabschnitte, radiale Unterabschnitte, inselförmige Unterabschnitte, etc.

Eine Zweikomponentenfaser mit einer "Kern/Hüll-Struktur" hat einen Querschnitt, der Folgendes umfasst: zwei diskrete Abschnitte, von denen jeder aus einem Polymer oder einer Polymermischung besteht, worin das Hüllpolymer oder die Hüllpolymermischkomponente um das Kernpolymer oder die Kernpolymermischungskomponente herum angeordnet ist.

Das Flächengewicht von Vliesmaterialien wird in der Regel in Gramm pro Quadratmeter (g/m²) angegeben.

In einer bevorzugten Ausführungsform umfasst die Flüssigkeitsaufnahmeschicht Mehrkomponentenfasern, insbesondere Zweikomponentenfasern, weiter insbesondere Polyester aufweisende Zweikomponentenfasern, sogenannte Bico/PES-Fasern. Die Mehrkomponentenfasern, insbesondere die Zweikomponentenfasern, weisen bevorzugt einen kreisfömigen oder dreilappigen Querschnitt auf.

Bevorzugte Kombinationen von Komponenten in Zweikomponentenfasern sind Polyethylenterephthalat (PET) / Polyethylen (PE), PET / Polypropylen (PP), PET / Polyester-Copolymere (CoPET), Polymilchsäure (PLA) / Polylactid-Copolymere (COPLA), PLA / PE und PLA / PP.

Als eine Alternative ist es im Fachgebiet bekannt, ein Material aus chemisch modifizierten Zellulosefasern, beispielsweise quervernetzten Zellulosefasern, als Flüssigkeitsaufnahmeschicht zu verwenden.

Die Flüssigkeitsaufnahmeschicht kann auch andere Materialien wie perforierte Folien oder Schäume oder dergleichen umfassen oder daraus bestehen.

Die Flüssigkeitsaufnahmeschicht kann die mindestens eine Speicherschicht des Absorptionskörpers im Wesentlichen vollständig oder nur teilweise überfangen, mithin im Wesentlichen dieselbe flächenhafte Erstreckung oder eine zumindest bereichsweise geringere Erstreckung als die mindestens eine Speicherschicht und/oder die dem Backsheet unmittelbar zugewandte Schicht des Absorptionskörpers in Längs- und/oder Querrichtung des plan aufliegenden Inkontinenzartikels aufweisen. Bevorzugt überfängt die Flüssigkeitsaufnahmeschicht die mindestens eine Speicherschicht und/oder die dem Backsheet unmittelbar zugewandte Schicht des Absorptionskörpers zu 5-100%, insbesondere zu 10-90%, weiter insbesondere zu 15-80%, weiter insbesondere zu 20-70% ihrer flächenhaften Erstreckung.

Vorzugsweise ist die Flüssigkeitsaufnahmeschicht zumindest in einem Bereich angeordnet in welchem die Miktionsflüssigkeit in Gebrauch auf den Inkontinenzartikel trifft. Insbesondere erstreckt sich die Flüssigkeitsaufnahmeschicht über und im Bereich einer Quermittelachse des Absorptionskörpers.

In einer bevorzugten Ausführungsform ist das pH-Regulationsmittel im Wesentlichen zwischen der Flüssigkeitsaufnahmeschicht und einer dem Backsheet zugewandten Absorptionskörperschicht positioniert. Insbesondere sind zumindest 70 Gewichtsprozent, weiter insbesondere 80 Gewichtsprozent, weiter insbesondere zumindest 90 Gewichtsprozent, weiter insbesondere zumindest 95 Gewichtsprozent des pH-Regulationsmittels zwischen der Flüssigkeitsaufnahmeschicht und der dem Backsheet zugewandten Absorptionskörperschicht positioniert.

Vorzugsweise berührt das pH-Regulationsmittel im trockenen Zustand des Inkontinenzartikels nicht das Topsheet, insbesondere sind zumindest 70 Gewichtsprozent, weiter insbesondere 80 Gewichtsprozent, weiter insbesondere zumindest 90 Gewichtsprozent, weiter insbesondere zumindest 95 Gewichtsprozent des pH-Regulationsmittels zwischen einer dem Topsheet zugewandten Schicht des Absorptionskörpers, insbesondere der Flüssigkeitsaufnahmeschicht und der in Richtung des Backsheets nächstliegenden Schicht des Absorptionskörpers positioniert.

Daraus ergibt sich in vorteilhafter Weise, dass das pH-Regulationsmittel bei Benutzung des Inkontinenzartikels auf einer der Haut des Benutzers abgewandten Oberseite der Flüssigkeitsaufnahmeschicht oder einer anderen Absorptionskörperschicht angeordnet ist und insbesondere nicht mit der Haut des Benutzers in direktem Kontakt steht.

Alternativ kann das pH-Regulationsmittel im Wesentlichen zwischen einer dem Topsheet zugewandten Oberseite der Flüssigkeitsaufnahmeschicht und der dem Backsheet zugewandten Absorptionskörperschicht, insbesondere im Wesentlichen innerhalb der Flüssigkeitsaufnahmeschicht positioniert sein.

Es erweist sich dabei als vorteilhaft, wenn der pH-Wert auf einer der Haut zugewandten Oberseite des Inkontinenzartikels einen Wert von 4.8-6.5, insbesondere 5.0-6.2, weiter insbesondere 5.2-6.0, weiter insbesondere 5.3-5.8, weiter insbesondere 5.4-5.6 beträgt. Bevorzugt ist, wenn der Inkontinenzartikel über mindestens zwei, insbesondere mindestens drei Miktionsereignisse hinweg, einen hautfreundlichen pH-Wert aufrechterhält.

Eine vorteilhafte Weiterentwicklung der oben beschriebenen Ausführungsformen ist eine Anordnung von Inkontinenzartikeln, wobei die Anordnung mindestens zwei, insbesondere mindestens drei, weiter insbesondere mindestens vier, weiter insbesondere mindestens fünf, weiter insbesondere mindestens sechs Inkontinenzartikel umfasst, wobei zumindest ein erster Inkontinenzartikel und ein zweiter Inkontinenzartikel der Anordnung sich in mindestens einem Merkmal, ausgewählt aus der Gruppe Größe, Absorptionskapazität nach ISO 11948-1 (1996), Menge an pH-Regulationsmittel in g/m², Gesamtmenge an pH-Regulationsmittel pro Inkontinenzartikel, Anzahl der Absorptionskörperschichten, unterscheiden

Die Gesamtmenge an pH-Regulationsmittel pro Inkontinenzartikel, der insbesondere zur Benutzung durch Erwachsene vorgesehen ist, beträgt vorzugsweise 0,2 bis 3,0 g, weiter vorzugsweise 0,4 bis 2,5 g, weiter vorzugsweise 0,6 bis 2,0 g.

Inkontinenzartikel einer bevorzugten Anordnung unterscheiden sich dann in der Menge an pH-Regulationsmittel in g/m oder in der Gesamtmenge an pH-Regulationsmittel pro Inkontinenzartikel oder Absorptionskapazität nach ISO 11948-1 (1996) voneinander, wenn der erste Inkontinenzartikel einen jeweiligen Wert aufweist, der mindestens 20%, insbesondere mindestens 30%, weiter insbesondere mindestens 40%, weiter insbesondere mindestens 50%, weiter insbesondere mindestens 75%, weiter insbesondere mindestens 100% höher ist als der jeweilige Wert des zweiten Inkontinenzartikels.

Eine Anordnung ergibt sich durch die sinnfällige Herrichtung der zur Anordnung gehörenden Inkontinenzartikel, insbesondere durch die Beziehung oder das Verhältnis der Artikel zueinander. Diese erfolgt entweder durch Darbietung in einer gemeinsamen Verpackungseinheit und/oder bevorzugt durch die Anbringung von Kennzeichnungen auf den Inkontinenzartikeln und/oder deren Verpackung und/oder die Darbietung in räumlicher oder inhaltlicher Nähe zueinander, die die Zugehörigkeit zu einer Anordnung kenntlich machen. Die die Anordnung bildenden Inkontinenzartikel stammen bevorzugt von ein und demselben Hersteller.

Die eine Anordnung bildenden Inkontinenzartikel weisen bevorzugt dieselbe Produktkennzeichnung auf, wie eben Markennamen und/oder Sub-Markennamen.

Eine Anordnung aus Inkontinenzartikeln einer ersten Ausprägung eines Merkmals und Inkontinenzartikeln einer zweiten Ausprägung eines Merkmals, wie beispielsweise einer ersten Größe und einer zweiten Größe, wird verstanden als zumindest jeweils einem Vertreter davon und schließt eine Mehrzahl der Inkontinenzartikel einer ersten Ausprägung eines Merkmals und/oder der Inkontinenzartikel einer zweiten Ausprägung eines Merkmals ein oder solche enthaltenden Packungen und Verpackungseinheiten.

Weiterhin kann die Anordnung mindestens zwei, insbesondere mindestens drei, weiter insbesondere mindestens vier, weiter insbesondere mindestens fünf, weiter insbesondere mindestens sechs Inkontinenzartikel umfassen, wobei zumindest ein erster Inkontinenzartikel und ein zweiter Inkontinenzartikel der Anordnung verschiedene Inkontinenzartikel ausgewählt aus der Gruppe Inkontinenzvorlage, Inkontinenzwindel offenen Typs mit Verschlusssystemen, Inkontinenzwindel geschlossenen Typs, Krankenunterlage, sind.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung und Beispielen. In der Zeichnung zeigt:
**Figur 1**
   schematische Darstellung in Draufsicht einer Inkontinenzwindel offenen Typs mit Verschlusselementen als Ausführungsbeispiel für einen erfindungsgemäßen Inkontinenzartikel
**Figur 2a**
   schematisch, einen Querschnitt durch einen Inkontinenzartikel mit einer Speicherschicht mit SAP-Material, einer Flüssigkeitsaufnahmeschicht und dazwischen angeordnetem pH-Regulationsmittel
**Figur 2b**
   schematisch, einen Querschnitt durch einen Inkontinenzartikel mit zwei Speicherschichten, SAP-Material, einer Flüssigkeitsaufnahmeschicht und dazwischen angeordnetem pH-Regulationsmittel
**Figur 3**
   schematisch, Positionen von Messpunkten zur Oberflächenmessung von pH-Werten relativ zu einer Flüssigkeitsaufnahmeschicht

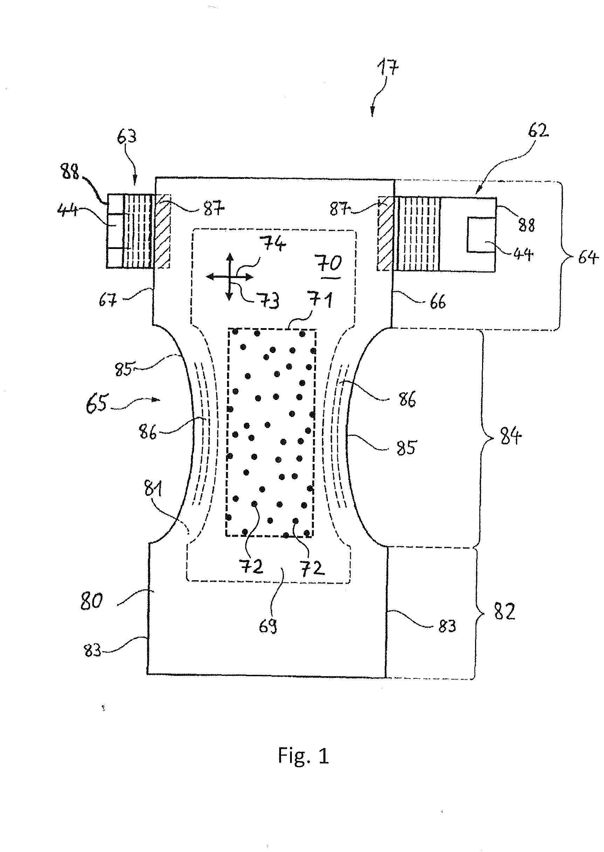

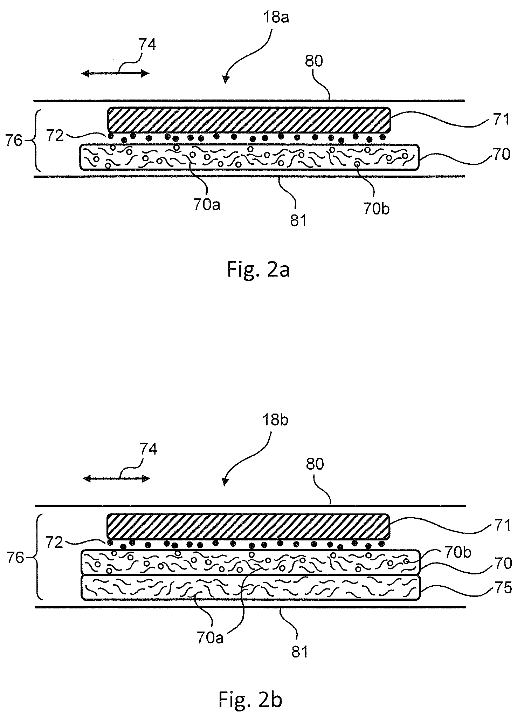

Die **Figur 1** zeigt, nicht maßstäblich, sondern schematisch einen insgesamt mit dem Bezugszeichen 17 bezeichneten erfindungsgemäßen Inkontinenzartikel, beispielhaft eine Inkontinenzwindel offenen Typs mit Verschlusselementen in der sogenannten T-Form für Erwachsene. Die Inkontinenzwindel 17 umfasst einen insgesamt mit dem Bezugszeichen 65 bezeichneten Hauptteil (Chassis) mit einem Körperflüssigkeiten absorbierenden Absorptionskörper 69. Der Absorptionskörper 69 umfasst in diesem Ausführungsbeispiel mindestens eine Speicherschicht 70, die SAP-Material (in **Figur 1** nicht gezeigt) enthält und eine Flüssigkeitsaufnahmeschicht 71. Die erfindungsgemäße Inkontinenzwindel 17 enthält ein pH-Regulationsmittel 72, das im Wesentlichen zwischen der Flüssigkeitsaufnahmeschicht 71 und der mindestens einen Speicherschicht 70 des Absorptionskörpers angeordnet ist, wie in **Figuren 2a und 2b** näher beschrieben. Der Absorptionskörper 69 ist zwischen zwei Flachmaterialien, und zwar einem zumindest bereichsweise flüssigkeitsdurchlässigen Topsheet 80 und einem im Wesentlichen flüssigkeitsundurchlässigen Backsheet 81 des Windelhauptteils 65 angeordnet.

Bei der Inkontinenzwindel 17 ist eine Längsrichtung 73 und eine Querrichtung 74 der Inkontinenzwindel 17 unterscheidbar, wobei letztere im angelegten Zustand der Inkontinenzwindel einer Hüftumfangsrichtung des Benutzers entspricht. Der Hauptteil 65 umfasst einen Vorderbereich 82 mit vorderen seitlichen Längsrändern 83, einen Rückenbereich 64 mit einem ersten hinteren seitlichen Längsrand 66 und einem zweiten hinteren seitlichen Längsrand 67 und einen dazwischen angeordneten Schrittbereich 84. An einem jeweiligen Längsrand 85 des Schrittbereichs 84 angrenzend weist der Hauptteil 65 je einen elastifizierten Abschnitt, mithin einen elastifizierten Beinöffnungsabschnitt 86 auf. Diese elastifizierten Beinöffnungsabschnitte 86 sind im dargestellten Fall gebildet durch zwischen Topsheet 80 und Backsheet 81 verlaufende und in vorgespanntem Zustand an Topsheet 80 und/oder Backsheet 81 fixierte elastische Fäden, die bogenförmig gekrümmt, mithin zumindest mit einer Komponente in Längsrichtung 73 der Inkontinenzwindel 17 orientiert sind.

Bei der T-förmigen Inkontinenzwindel 17 ist im Rückenbereich 64 des Hauptteils 65 in Querrichtung 74 der Inkontinenzwindel 17 ein seitlich über den ersten hinteren seitlichen Längsrand 66 hinaus erstrecktes erstes elastisches Windelseitenteil 62 und ein seitlich über den zweiten hinteren seitlichen Längsrand 67 hinaus erstrecktes zweites elastisches Windelseitenteil 63 vorgesehen, die im Bereich der hinteren seitlichen Längsränder 66 bzw. 67 in einem Überlappungsbereich 87 unlösbar an den Rückenbereich 64 des Hauptteils 65 angefügt sind. Im Vorderbereich 82 sind hingegen keine Windelseitenteile vorgesehen.

In einer alternativen Ausführungsvariante kann die Inkontinenzwindel auch als H-förmige Inkontinenzwindel ausgebildet sein, wobei dann zusätzlich im Vorderbereich und bevorzugt beidseitig Windelseitenteile ausgebildet sind, wie das beispielsweise in WO2005102241A1 gezeigt ist. Als weitere Ausführungsvariante ist auch eine Inkontinenzwindel des geschlossenen Typs denkbar, wobei solchen Falls vorzugsweise im Vorderbereich und im Rückenbereich jeweils ein Bauch- bzw. Rückenteil angefügt ist, welche an jeweiligen seitlichen Längsrändern des Bauch- und Rückenteils miteinander gefügt sein derart, dass die Inkontinenzwindel in Hüftumfangsrichtung ringförmig geschlossen ist, wie beispielsweise in WO2013171068A1 gezeigt. Als weitere Ausführungsvariante kann der Inkontinenzartikel 17 als Krankenunterlage oder Inkontinenzvorlage ausgebildet sein.

Das erste elastische Windelseitenteil 62 und das zweite elastische Windelseitenteil 63 der T-förmigen Inkontinenzwindel 17 haben im Bereich ihres in Querrichtung 74 der Inkontinenzwindel 17 freien Endes 88 jeweils wenigstens ein Verschlusselement 44. Das Verschlusselement 44 ist in Form einer vorzugsweise rechteckigen Lasche ausgebildet und herstellerseitig auf sich selbst eingefaltet. In der Gebrauchssituation lässt sich das Verschlusselement 44 öffnen, das heißt wieder auffalten, um die Inkontinenzwindel 17 an einen Benutzer anzulegen, wobei das erste elastische Windelseitenteil 62 und das zweite elastische Windelseitenteil 63 in Überlappung mit dem Vorderbereich 82 des Hauptteils 65 gebracht werden und die Verschlusselemente 44 lösbar haftend auf der vom Benutzer abgewandten Seite des Vorderbereichs 82 des Hauptteils 65 festgelegt werden.

Die **Figuren 2a und 2b** zeigen in beispielhafter Weise schematisch, nicht maßstäblich, jeweils einen Querschnitt durch je einen Inkontinenzartikel 18a, 18b, beispielsweise eine Inkontinenzwindel des offenen Typs mit Verschlusssystemen (wie in **Figur 1** näher beschrieben) oder eine Inkontinenzwindel des geschlossenen Typs oder eine Inkontinenzvorlage oder eine Krankenunterlage. Die Inkontinenzartikel 18a, 18b weisen ein flüssigkeitsdurchlässiges Topsheet 80, ein im Wesentlichen (mithin in Gebrauch) flüssigkeitsundurchlässiges Backsheet 81, und einen dazwischen angeordneten Absorptionskörper 76 auf. Der Absorptionskörper 76 des Inkontinenzartikels 18a **(****Figur 2a****)** weist eine Speicherschicht 70 mit Fasermaterial 70a, wie beispielsweise Zellstofffasern oder Kunststofffasern, und SAP-Material 70b, eine Flüssigkeitsaufnahmeschicht 71 und ein dazwischen angeordnetes pH-Regulationsmittel 72 auf. Die Flüssigkeitsaufnahmeschicht 71 bildet eine dem Topsheet 80 unmittelbar zugewandte Schicht des Absorptionskörpers 76. Die Speicherschicht 70 ist dem Backsheet unmittelbar zugewandt und ist im Wesentlichen frei von dem pH-Regulationsmittel 72. Der Absorptionskörper des Inkontinenzartikels 18b **(****Figur 2b****)** weist zusätzlich eine weitere Schicht, nämlich eine dem Backsheet 81 unmittelbar zugewandte Schicht 75 des Absorptionskörpers 76 auf, die im Wesentlichen aus Fasermaterial 70a, wie beispielsweise Zellstofffasern oder Kunststofffasern, besteht und zwischen der Speicherschicht 70 und dem Backsheet 81 angeordnet ist. Die dem Backsheet 81 unmittelbar zugewandte Schicht 75 ist im Wesentlichen frei von pH-Regulationsmittel 72.

In den in **Figuren 2a und 2b** dargestellten Ausführungsbeispielen umfasst das pH-Regulationsmittel 72 partikelförmiges Mononatriumcitrat, Typ "Fine Granular F3500" von Jungbunzlauer. Dieses Mononatriumcitrat weist zu 59 Gewichtsprozent eine Partikelgröße von 80 bis 355 µm und eine Reinheit von mindestens 99 % auf. Die Menge an pH-Regulationsmittel 72 beträgt in diesem Beispiel 30 g/m². Als pH-Regulationsmittel kann im Prinzip jedes dem Fachmann als geeignet erscheinende und der Beschreibung der Erfindung entsprechende Mononatriumcitrat verwendet werden.

Das SAP-Material 70b ist in den dargestellten Fällen partikelförmig ausgebildet. In einer denkbaren Variante kann das SAP-Material faserförmig oder blatt- oder schaumförmig ausgebildet sein.

Die beiden **Figuren 2a und 2b** zeigen den Querschnitt durch die Inkontinenzartikel 18a bzw. 18b jeweils in Querrichtung 74, im Falle einer Inkontinenzwindel 17 beispielsweise im Schrittbereich (Bezugszeichen 84 in **Figur 1****,** ohne Darstellung der elastifizierten Beinöffnungsabschnitte 86).

Die Flüssigkeitsaufnahmeschicht 71 überfängt die Speicherschicht 70 des Absorptionskörpers 76 nur teilweise und weist vorliegend eine geringere Erstreckung als die Speicherschicht 70 **(****Figur 2a****)** oder die Speicherschicht 70 und die dem Backsheet 81 unmittelbar zugewandte Schicht 75 des Absorptionskörpers 76 **(****Figur 2b****)** in der Querrichtung 74 des plan aufliegenden Inkontinenzartikels 18 auf. Alternativ kann die Flüssigkeitsaufnahmeschicht die Speicherschicht und/oder die dem Backsheet 81 unmittelbar zugewandte Schicht 75 des Absorptionskörpers 76 vollständig überfangen. Die Speicherschicht 70 ist in diesem Beispiel **(****Figur 2b****)** in der Querrichtung 74 im Wesentlichen gleich erstreckt wie die dem Backsheet 81 unmittelbar zugewandte Schicht 75 des Absorptionskörpers 76. Die Speicherschicht 70 und die dem Backsheet 81 unmittelbar zugewandte Schicht 75 des Absorptionskörpers 76 können aber auch unterschiedlich erstreckt sein.

### Beispiel 1: pH-Regulation durch Mononatriumcitrat oder Dinatriumcitrat im Vergleich zu Citronensäure

1A: Eine Inkontinenzwindel für Erwachsene wurde mit einem pH-Regulationsmittel bestehend aus Mononatriumcitrat (erste Inkontinenzwindel 1A, erfindungsgemäß) hergestellt. Die Inkontinenzwindel 1A wies die folgenden Komponenten in der angegebenen Anordnung auf:
- Topsheet: SMS-Vliesmaterial, 12 g/m², Typ 3000063 von Avgol LTD
- Flüssigkeitsaufnahmeschicht: Vliesmaterial kardiert und thermisch verbunden ("airthroughbonded"), Bico/PES, 40 g/m², Abmessungen 90x270 mm (0,0243 m²), Typ 11040WC0A von Berry
- pH-Regulationsmittel: 25 g/m² Mononatriumcitrat, Reinheit >95%, nicht hydriert, Gesamtmenge pro Inkontinenzartikel 0,6 g, errechnete molare Gesamtmenge an Mononatriumcitrat pro Inkontinenzartikel 0,003 Mol, Katalognummer 21533 von Thermo Fisher Scientific
- Speicherschicht: 21,5 g Zellstofffasern ("fluff"), Typ CoosAbsorb S von Resolute; gemischt mit 13,5 g partikelförmigem SAP-Material (Typ SXM 9791 von Evonik)
- Dem Backsheet zugewandte Schicht des Absorptionskörpers: 34,8 g Zellstofffasern ("fluff"), Typ CoosAbsorb S von Resolute
- Backsheet: Vlies-Folienlaminat, 18 g/m², Hyfol PE soft Textile Typ 14202 von RKW

1B: Eine weitere Inkontinenzwindel für Erwachsene wurde mit einem pH-Regulationsmittel bestehend aus Dinatriumcitrat (zweite Inkontinenzwindel 1B, zum Vergleich, erfindungsgemäß) hergestellt. Die Inkontinenzwindel 1B wies die folgenden Komponenten in der angegebenen Anordnung auf:
- Topsheet: SMS-Vliesmaterial, 12 g/m², Typ 3000063 von Avgol LTD
- Flüssigkeitsaufnahmeschicht: Vliesmaterial kardiert und thermisch verbunden ("airthroughbonded"), Bico/PES, 40 g/m², Abmessungen 90x270 mm (0,0243 m²), Typ 11040WC0A von Berry
- pH-Regulationsmittel: 31 g/m² Dinatriumcitrat, Reinheit 99%, Hydratform (Dinatriumhydrogencitratsesquihydrat), Gesamtmenge pro Inkontinenzartikel 0,75 g, errechnete molare Gesamtmenge an Dinatriumcitrat pro Inkontinenzartikel 0,003 Mol, Katalognummer 25024 von Thermo Fisher Scientific
- Speicherschicht: 21,5 g Zellstofffasern ("fluff"), Typ CoosAbsorb S von Resolute; gemischt mit 13,5 g partikelförmigem SAP-Material (Typ SXM 9791 von Evonik)
- Dem Backsheet zugewandte Schicht des Absorptionskörpers: 34,8 g Zellstofffasern ("fluff"), Typ CoosAbsorb S von Resolute
- Backsheet: Vlies-Folienlaminat, 18 g/m², Hyfol PE soft Textile Typ 14202 von RKW

1C: Eine weitere Inkontinenzwindel für Erwachsene wurde mit einem pH-Regulationsmittel bestehend aus Citronensäure (dritte Inkontinenzwindel 1C, zum Vergleich, nicht erfindungsgemäß) hergestellt. Die Inkontinenzwindel 1C wies die folgenden Komponenten in der angegebenen Anordnung auf:
- Topsheet: SMS-Vliesmaterial, 12 g/m², Typ 3000063 von Avgol LTD
- Flüssigkeitsaufnahmeschicht: Vliesmaterial kardiert und thermisch verbunden ("airthroughbonded"), Bico/PES, 40 g/m², Abmessungen 90x270 mm (0,0243 m²), Typ 11040WC0A von Berry
- pH-Regulationsmittel: 26 g/m² Citronensäure, Reinheit mindestens 99,5%, nicht hydriert, Gesamtmenge pro Inkontinenzartikel 0,625 g, errechnete molare Gesamtmenge an Citronensäure pro Inkontinenzartikel 0,003 Mol, Artikelnummer 471A1F, "citric acid anhydrous pharm" von Barcelonesa
- Speicherschicht: 21,5 g Zellstofffasern ("fluff"), Typ CoosAbsorb S von Resolute; gemischt mit 13,5 g partikelförmigem SAP-Material (Typ SXM 9791 von Evonik)
- Dem Backsheet zugewandte Schicht des Absorptionskörpers: 34,8 g Zellstofffasern ("fluff"), Typ CoosAbsorb S von Resolute
- Backsheet: Vlies-Folienlaminat, 18 g/m², Hyfol PE soft Textile Typ 14202 von RKW

Das pH-Regulationsmittel war in der ersten 1A und zweiten 1B und dritten Inkontinenzwindel 1C zwischen der Flüssigkeitsaufnahmeschicht und der Speicherschicht angeordnet.

Zur besseren Vergleichbarkeit der pH-Regulation in der ersten 1A und zweiten 1B und dritten Inkontinenzwindel 1C wurde die jeweilige Gesamtmenge an pH-Regulationsmittel pro Inkontinenzartikel so gewählt, dass jeweils eine im Wesentlichen gleiche molare Gesamtmenge von 0,003 mol pro Inkontinenzartikel an dem jeweiligen pH-Regulationsmittel vorlag (siehe **Tabelle 1).**

**Tabelle 1: In Beispiel 1 verwendete pH-Regulationsmittel**

| | 1A (mit MNC) | 1B (mit DNC) | 1C (mit CS) |
|---|---|---|---|
| Kristallwasser | nicht hydriert | Sesquihydrat | nicht hydriert |
| Molarität [g/mol] | 214,11 | 263,1 | 192,13 |
| Gesamtmenge pro Inkontinenzartikel [g] | 0,6 | 0,75 | 0,625 |
| von pH-Regulationsmittel überfangene Fläche [m²] | 0,0243 | 0,0243 | 0,0243 |
| Menge an pH-Regulationsmittel [g/m²] | 25 | 31 | 26 |
| molare Gesamtmenge pro Inkontinenzartikel [mol] | 0,003 | 0,003 | 0,003 |
| MNC, Mononatriumcitrat; DNC, Dinatriumcitrat; CS, Citronensäure | | | |

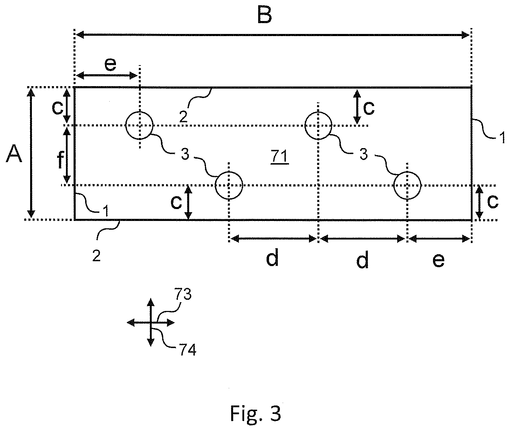

Wie unten im Abschnitt zur Testmethode näher beschrieben wurden drei aufeinander folgende Miktionsereignisse mit einer Urinersatzflüssigkeit simuliert und die pH-Werte an einer jeweiligen äußeren Oberseite des Topsheets des ersten und zweiten und dritten Inkontinenzartikels im Bereich der Flüssigkeitsaufnahmeschicht 71 gemessen. **Figur 3** zeigt die Positionen von vier Messpunkten 3 relativ zur Flüssigkeitsaufnahmeschicht 71 des ersten und zweiten und dritten Inkontinenzartikels (Blickrichtung auf das Topsheet). Eine Quererstreckung A der Flüssigkeitsaufnahmeschicht 71 betrug jeweils 90 mm. Eine Längserstreckung B der Flüssigkeitsaufnahmeschicht 71 betrug 270 mm. Es wurde an allen vier Messpunkten 3 der pH-Wert gemessen und aus den jeweils 4 Messwerten pro Zeitpunkt ein Durchschnittswert errechnet, der in Tabelle 3 wiedergegeben ist. Aus den nach jeweils einer simulierten Miktion erhaltenen pH-Durchschnittswerten wurde zudem ein Mittelwert und eine jeweils dazugehörige Standardabweichung errechnet und ebenfalls in Tabelle 3 dargestellt. Die Messpunkte 3 waren in Längsrichtung 73 der jeweiligen Flüssigkeitsaufnahmeschicht derart verteilt, dass ein Abstand e zwischen einem in Längsrichtung 73 einem jeweiligen Querrand 1 der Flüssigkeitsaufnahmeschicht 71 am nächsten gelegenen Messpunktes 3 und dem jeweiligen in Längsrichtung 73 nächstgelegenen Querrand 1 45 mm betrug. Ein Abstand d zwischen zwei in Längsrichtung 73 aufeinander folgenden Messpunkten 3 betrug in diesem Beispiel 60 mm.

Ein Abstand c zwischen einem in Querrichtung 74 einem jeweiligen Längsrand 2 der jeweiligen Flüssigkeitsaufnahmeschicht 71 am nächsten gelegenen Messpunktes 3 und dem jeweiligen in Querrichtung 74 nächstgelegenen Längsrand 2 betrug 25 mm. Ein Abstand f zwischen zwei in Querrichtung 74 aufeinander folgenden Messpunkten 3 betrug in diesem Beispiel 40 mm.

Jeweilige Flüssigkeitsvolumina der drei simulierten Miktionen sind **Tabelle 2** zu entnehmen.

**Tabelle 2: In Beispiel 1 eingesetzte Volumina der Urinersatzflüssigkeit**

| | Flüssigkeitsvolumen [ml] | Anteil an der maximalen Absorptionskapazität [%] |
|---|---|---|
| Erste simulierte Miktion | 300 | 45 |
| Zweite simulierte Miktion | 150 | 22,5 |
| Dritte simulierte Miktion | 150 | 22,5 |
| Summe | 600 | 90 |

Die zu den verschiedenen Zeitpunkten an den beschriebenen vier Messpunkten 3 gemessenen pH-Werte sind **Tabelle 3** zu entnehmen.

**Tabelle 3: pH-Wert-Regulation, Mononatriumcitrat und Dinatriumcitrat im Vergleich zu Citronensäure, einzelnen Zeitpunkten zugeordnete pH-Werte sind Durchschnittswerte aus je vier Messwerten**

| | erste simulierte Miktion | | | zweite simulierte Miktion | | | dritte simulierte Miktion | | |
|---|---|---|---|---|---|---|---|---|---|
| Zeit [min]* | 1A (mit MNC) | 1B (mit DNC) | 1C (mit CS) | 1A (mit MNC) | 1B (mit DNC) | 1C (mit CS) | 1A (mit MNC) | 1B (mit DNC) | 1C (mit CS) |
| 1 | 5,2 | 5,0 | 4,3 | 5,4 | 5,7 | 5,4 | 5,3 | 5,7 | 5,4 |
| 3 | 5,3 | 5,3 | 4,0 | 5,4 | 5,6 | 5,3 | 5,5 | 5,7 | 5,3 |
| 5 | 5,2 | 5,3 | 4,3 | 5,5 | 5,5 | 5,3 | 5,4 | 5,7 | 5,4 |
| 10 | 5,3 | 5,1 | 4,5 | 5,5 | 5,4 | 5,3 | 5,5 | 5,8 | 5,4 |
| 15 | 5,2 | 5,3 | 4,1 | 5,4 | 5,7 | 5,4 | 5,5 | 5,7 | 5,6 |
| 20 | 5,1 | 5,3 | 4,3 | 5,5 | 5,6 | 5,4 | 5,5 | 5,6 | 5,7 |
| MW | 5,2 | 5,2 | 4,3 | 5,5 | 5,6 | 5,4 | 5,5 | 5,7 | 5,5 |
| SD | 0,1 | 0,1 | 0,2 | 0,1 | 0,1 | 0,1 | 0,1 | 0,1 | 0,1 |
| MNC, Mononatriumcitrat; DNC, Dinatriumcitrat; CS, Citronensäure MW, Mittelwert der in der jeweiligen Spalte vorangehenden pH-Durchschnittswerte der in der Spaltenüberschrift angegebenen Miktion; SD, Standardabweichung | | | | | | | | | |
| * nach der jeweiligen simulierten Miktion | | | | | | | | | |

Die dritte Inkontinenzwindel 1C mit Citronensäure ergab nach der ersten simulierten Miktion pH-Werte von 4,0 bis 4,5 und lag dabei mit einem Mittelwert von pH 4,3 ± SD=0,2 deutlich unterhalb des hautfreundlichen Bereiches. Erst mit der zweiten und dritten simulierten Miktion zwei und fünf Stunden nach Beginn des Versuches stellte sich ein hautfreundlicher pH-Wert im Bereich von 5,4 bis 5,5 ein (Mittelwerte pH 5,4 ± SD=0,1 und pH 5,5 ± SD=0,1).

Die zweite Inkontinenzwindel 1B mit Dinatriumcitrat ergab bereits nach der ersten simulierten Miktion hautfreundliche pH-Werte von pH 5,0 bis 5,3 (Mittelwert pH 5,2 ± SD=0,1) und stieg nach der zweiten und dritten simulierten Miktion nur leicht an (Mittelwerte pH 5,6 ± SD=0,1 und pH 5,7 ± SD=0,1).

Die erste Inkontinenzwindel 1A mit Mononatriumcitrat hielt ebenfalls über drei simulierte Miktionsereignisse hinweg einen relativ konstanten hautfreundlichen pH-Wert von 5,1 bis 5,5 aufrecht und hielt insbesondere nach der zweiten und dritten simulierten Miktion den pH-Wert konstant bei pH 5,4 bis 5,5 (Mittelwerte jeweils pH 5,5 ± SD=0,1).

Insbesondere war überraschend, dass sowohl die erste 1A als auch die zweite Inkontinenzwindel 1B nach der ersten simulierten Miktion einen ungefähr gleichen und hautfreundlichen pH-Wert (Mittelwert jeweils pH 5,2 ± SD=0,1), dabei jedoch einen deutlich höheren pH-Wert als die dritte Inkontinenzwindel 1C mit Citronensäure (Mittelwert pH 4,3 ± SD=0,2) aufwiesen, obwohl alle drei Inkontinenzwindeln eine im Wesentlichen gleiche molare Gesamtmenge an pH-Regulationsmittel aufwiesen (jeweils 0,003 mol). Weiter überraschend war, dass die pH-Werte sowohl der ersten 1A als auch der zweiten Inkontinenzwindel 1B nach der zweiten und dritten simulierten Miktion nicht oder nicht wesentlich stärker anstiegen als die pH-Werte der Inkontinenzwindel 1C mit Citronensäure (Mittelwerte **Tabelle 3).**

Die erste Inkontinenzwindel 1A mit Mononatriumcitrat ergab über die Gesamtdauer des Versuchs hinweg eine nur wenig bessere pH-Regulation als die zweite Inkontinenzwindel 1B mit Dinatriumcitrat, bei im Wesentlichen gleicher molarer Gesamtmenge an pH-Regulationsmittel (jeweils 0,003 mol). Im Vergleich zu dem in diesem Beispiel verwendeten hydrierten Dinatriumcitrat kann daher bei Verwendung von Mononatriumcitrat gewichtsmäßig ungefähr 20% weniger pH-Regulationsmittel eingesetzt werden. Somit können sich durch die Wahl des pH-Regulationsmittels neben oben beschriebenen prozesstechnischen Vorteilen zusätzlich auch ökonomische und logistische Vorteile ergeben.

### Beispiel 2: pH-Regulation durch ein pH-Regulationsmittel bestehend aus Citronensäure und Trinatriumcitrat

2A: Eine Inkontinenzwindel für Erwachsene wurde mit einem im Stand der Technik (WO2012121932A1) bekannten pH-Regulationsmittel bestehend aus einer Mischung aus Trinatriumcitrat und Citronensäure hergestellt, wobei das Mischungsverhältnis 6,2:1 (w/w) betrug (vierte Inkontinenzwindel 2A, zum Vergleich, nicht erfindungsgemäß). Die Inkontinenzwindel 2A wies die folgenden Komponenten in der angegebenen Anordnung auf:
- Topsheet: SMS-Vliesmaterial, 12 g/m², Typ 3000063 von Avgol LTD
- Flüssigkeitsaufnahmeschicht: Vliesmaterial kardiert und thermisch verbunden ("airthroughbonded"), Bico/PES, 40 g/m², Abmessungen 90x270 mm (0,0243 m²), Typ 11040WC0A von Berry
- pH-Regulationsmittel: 31 g/m² (Gesamtmenge pro Inkontinenzartikel 0,75 g) Trinatriumcitrat: Citronensäure 6,2:1 (w/w)
- Speicherschicht: 21,5 g Zellstofffasern ("fluff"), Typ CoosAbsorb S von Resolute; gemischt mit 13,5 g partikelförmigem SAP-Material (Typ SXM 9791 von Evonik)
- Dem Backsheet zugewandte Schicht des Absorptionskörpers: 34,8 g Zellstofffasern ("fluff"), Typ CoosAbsorb S von Resolute
- Backsheet: Vlies-Folienlaminat, 18 g/m², Hyfol PE soft Textile Typ 14202 von RKW

2B: Eine Inkontinenzwindel für Erwachsene wurde mit einem im Stand der Technik (WO2012121932A1) bekannten pH-Regulationsmittel bestehend aus einer Mischung aus Trinatriumcitrat und Citronensäure hergestellt, wobei das Mischungsverhältnis 7,2:1 (w/w) betrug (fünfte Inkontinenzwindel 2B, zum Vergleich, nicht erfindungsgemäß). Die Inkontinenzwindel 2B wies die folgenden Komponenten in der angegebenen Anordnung auf:
- Topsheet: SMS-Vliesmaterial, 12 g/m², Typ 3000063 von Avgol LTD
- Flüssigkeitsaufnahmeschicht: Vliesmaterial kardiert und thermisch verbunden ("airthroughbonded"), Bico/PES, 40 g/m², Abmessungen 90x270 mm (0,0243 m²), Typ 11040WC0A von Berry
- pH-Regulationsmittel: 31 g/m² (Gesamtmenge pro Inkontinenzartikel 0,75 g) Trinatriumcitrat: Citronensäure 7,2:1 (w/w)
- Speicherschicht: 21,5 g Zellstofffasern ("fluff"), Typ CoosAbsorb S von Resolute; gemischt mit 13,5 g partikelförmigem SAP-Material (Typ SXM 9791 von Evonik)
- Dem Backsheet zugewandte Schicht des Absorptionskörpers: 34,8 g Zellstofffasern ("fluff"), Typ CoosAbsorb S von Resolute
- Backsheet: Vlies-Folienlaminat, 18 g/m², Hyfol PE soft Textile Typ 14202 von RKW

Das in der vierten 2A und fünften Inkontinenzwindel 2B eingesetzte Trinatriumcitrat wies eine Reinheit von >99% auf und lag als Dihydrat vor (Artikelnummer 27833.363 von VWR chemicals). Die Citronensäure lag als nicht hydrierte Form vor und wies eine Reinheit von mindestens 99,5% auf (Artikelnummer 471A1F, "citric acid anhydrous pharm" von Barcelonesa).

Das pH-Regulationsmittel war in der vierten 2A und fünften Inkontinenzwindel 2B zwischen der Flüssigkeitsaufnahmeschicht und der Speicherschicht angeordnet.

Wie in Bezug auf Beispiel 1 und im Abschnitt zur Testmethode näher beschrieben wurden drei aufeinander folgende Miktionsereignisse mit einer Urinersatzflüssigkeit simuliert und die pH-Werte an der jeweiligen äußeren Oberseite des Topsheets des vierten und fünften Inkontinenzartikels im Bereich der Flüssigkeitsaufnahmeschicht 71 gemessen. Figur 3 zeigt die Positionen der vier Messpunkte 3 relativ zur Flüssigkeitsaufnahmeschicht 71 des vierten und fünften Inkontinenzartikels aus der Blickrichtung des Topsheets. Die Quererstreckung A der Flüssigkeitsaufnahmeschicht 71 betrug jeweils 90 mm. Die Längserstreckung B der Flüssigkeitsaufnahmeschicht 71 betrug 270 mm. Es wurde an allen vier Messpunkten 3 der pH-Wert gemessen und aus den jeweils 4 Messwerten pro Zeitpunkt ein Durchschnittswert errechnet, der in Tabelle 5 wiedergegeben ist. Aus den nach jeweils einer simulierten Miktion erhaltenen pH-Durchschnittswerten wurde zudem ein Mittelwert und die jeweils dazugehörige Standardabweichung errechnet und ebenfalls in **Tabelle 5** dargestellt. Die Messpunkte 3 waren in Längsrichtung 73 der jeweiligen Flüssigkeitsaufnahmeschicht derart verteilt, dass ein Abstand e zwischen einem in Längsrichtung 73 einem jeweiligen Querrand 1 der Flüssigkeitsaufnahmeschicht 71 am nächsten gelegenen Messpunktes 3 und dem jeweiligen in Längsrichtung 73 nächstgelegenen Querrand 1 45 mm betrug. Ein Abstand d zwischen zwei in Längsrichtung 73 aufeinander folgenden Messpunkten 3 betrug in diesem Beispiel 60 mm.

Ein Abstand c zwischen einem in Querrichtung 74 einem jeweiligen Längsrand 2 der jeweiligen Flüssigkeitsaufnahmeschicht 71 am nächsten gelegenen Messpunktes 3 und dem jeweiligen in Querrichtung 74 nächstgelegenen Längsrand 2 betrug 25 mm. Ein Abstand f zwischen zwei in Querrichtung 74 aufeinander folgenden Messpunkten 3 betrug in diesem Beispiel 40 mm.

Die Flüssigkeitsvolumina der drei simulierten Miktionen sind **Tabelle 4** zu entnehmen.

**Tabelle 4: In Beispiel 2 eingesetzte Volumina der Urinersatzflüssigkeit**

| | Flüssigkeitsvolumen [ml] | Anteil and der maximalen Absorptionskapazität [%] |
|---|---|---|
| Erste simulierte Miktion | 300 | 45 |
| Zweite simulierte Miktion | 150 | 22,5 |
| Dritte simulierte Miktion | 150 | 22,5 |
| Summe | 600 | 90 |

Die zu den verschiedenen Zeitpunkten an den beschriebenen Messpunkten 3 gemessenen pH-Werte sind **Tabelle 5** zu entnehmen.

**Tabelle 5: pH-Wert-Regulation, pH-Regulationsmittel bestehend aus Citronensäure und Trinatriumcitrat, einzelnen Zeitpunkten zugeordnete pH-Werte sind Durchschnittswerte aus je vier Messwerten**

| | erste simulierte Miktion | | zweite simulierte Miktion | | dritte simulierte Miktion | |
|---|---|---|---|---|---|---|
| Zeit [min]* | 2A (6,2:1) | 2B (7,2:1) | 2A (6,2:1) | 2B (7,2:1) | 2A (6,2:1) | 2B (7,2:1) |
| 1 | 5,7 | 5,6 | 5,8 | 5,7 | 5,9 | 5,8 |
| 3 | 5,5 | 5,7 | 5,8 | 5,8 | 5,9 | 5,9 |
| 5 | 5,7 | 5,7 | 5,8 | 5,8 | 5,8 | 5,9 |
| 10 | 5,6 | 5,6 | 5,9 | 5,8 | 5,8 | 5,8 |
| 15 | 5,6 | 5,7 | 5,9 | 5,8 | 5,9 | 5,8 |
| 20 | 5,6 | 5,7 | 5,9 | 5,8 | 5,9 | 5,8 |
| MW | 5,6 | 5,7 | 5,9 | 5,8 | 5,9 | 5,8 |
| SD | 0,1 | 0,1 | 0,1 | 0,0 | 0,1 | 0,1 |
| MW, Mittelwert der in der jeweiligen Spalte vorangehenden pH-Durchschnittswerte der in der Spaltenüberschrift angegebenen Miktion; SD, Standardabweichung | | | | | | |
| * nach der jeweiligen simulierten Miktion | | | | | | |

Die vierte und fünfte Inkontinenzwindel 2A und 2B mit dem bekannten pH-Regulationsmittel bestehend aus einer Mischung aus Trinatriumcitrat und Citronensäure in verschiedenen Mischungsverhältnissen ergaben nach der ersten simulierten Miktion pH-Werte von 5,5 bis 5,7. Nach der zweiten und dritten simulierten Miktion stieg der jeweilige pH-Wert in der vierten und fünften Inkontinenzwindel auf relativ hohe pH-Werte von 5,7 bis 5,9. Trotz unterschiedlicher Mischungsverhältnisse von Trinatriumcitrat und Citronensäure zeigten die vierte und fünfte Inkontinenzwindel 2A und 2B eine ungefähr gleiche pH-Regulation, so dass in diesem Beispiel keines der beiden Mischungsverhältnisse gegenüber dem jeweils anderen einen Vorteil bot.

In der Zusammenschau der beiden Beispiele 1 und 2 zeigt sich, dass das pH-Regulationsmittel umfassend Mononatriumcitrat der ersten Inkontinenzwindel 1A oder Dinatriumcitrat der zweiten Inkontinenzwindel 1B allen anderen der getesteten dritten 1C, vierten 2A und fünften Inkontinenzwindel 2B überlegen war, da die pH-Werte der ersten 1A und zweiten Inkontinenzwindel 1B über alle drei simulierten Miktionen hinweg innerhalb des hautfreundlichen Bereiches lagen, dabei insbesondere die pH-Werte innerhalb eines engen Bereiches relativ konstant blieben, und weiter insbesondere kein wesentlicher Abfall der pH-Werte nach der ersten Miktion und kein wesentlicher Anstieg auch über mehr als 5 Stunden hinweg mit insgesamt dreimaliger simulierter Miktion erfolgte.

### Testmethode pH-Wert-Messung an der Oberfläche eines Inkontinenzartikels:

Zur Durchführung einer pH-Wert-Messung an der Oberfläche eines Inkontinenzartikels, insbesondere auf einer äußeren Oberseite des Topsheets des Inkontinenzartikels, wird eine Urinersatzlösung verwendet. Die Urinersatzlösung ist eine nach ISO 11984-1 (1996) hergestellte 0,9 %ige (w/v) Lösung aus Natriumchlorid (NaCl) in destilliertem Wasser, welche mit Natriumhydroxid (NaOH) auf pH 6.8 eingestellt ist. Eine solche Lösung ist im allgemeinen Fachwissen seit langem bekannt und deren Herstellung gehört zu den Routinemethoden des Fachgebiets.

Der zu testende Inkontinenzartikel wird mit der Topsheet-Seite nach oben flach ausgebreitet, dabei gegebenenfalls vollständig aufgefaltet und gegebenenfalls auf einer Unterlage fixiert.

Der Test wird entsprechend der ISO 11984-1 (1996) bei einer Temperatur von 23 °C ± 2 °C und einer relativen Luftfeuchtigkeit von 50% ± 5% durchgeführt. Der zu testende Inkontinenzartikel und die Urinersatzflüssigkeit werden gemäß ISO 11984-1 (1996) entsprechend vorkonditioniert.

Um eine Benutzungssituation relativ realitätsnah abzubilden und die pH-Wert-Entwicklung oder die Pufferwirkung des pH-Regulationsmittels über eine längere Benutzungsdauer mit mehreren Miktionsereignissen zu verfolgen, werden wie nachfolgend beschrieben im Verlauf des Tests drei Miktionsereignisse über einen Zeitraum von fünf Stunden simuliert. Der Inkontinenzartikel wird hierbei insgesamt mit einem Flüssigkeitsvolumen beladen, das etwa 90% einer maximalen Absorptionskapazität des Inkontinenzartikels entspricht.

Die Volumina der ersten und zweiten und dritten simulierten Miktion sind daher so zu wählen, dass ein Gesamtvolumen der ersten und zweiten und dritten simulierten Miktion etwa 90% der maximalen Absorptionskapazität des Inkontinenzartikels entspricht. Dabei entspricht ein Volumen einer ersten simulierten Miktion 45% bis 50% der maximalen Absorptionskapazität des Inkontinenzartikels. Ein jeweiliges Volumen einer zweiten und einer dritten simulierten Miktion ist im Wesentlichen gleich und entspricht jeweils 20% bis 22,5% der maximalen Absorptionskapazität.

Falls die maximale Absorptionskapazität eines Inkontinenzartikels nicht bekannt ist, wird die maximale Absorptionskapazität anhand eines zweiten vergleichbaren Inkontinenzartikels, beispielsweise eines derselben Saugstärke und Größe und Produktart zuzuordnenden und gegebenenfalls derselben Packung zu entnehmenden Inkontinenzartikels wie der zu testende Inkontinenzartikel, vorab gemäß ISO 11984-1 (1996) bestimmt.

Ein Volumen der Urinersatzlösung, welches 50% der maximalen Absorptionskapazität des Inkontinenzartikels entspricht, wird innerhalb von 30 Sekunden zum Zweck der Simulierung einer ersten Miktion auf den trockenen Inkontinenzartikel gegossen und zwar derart, dass die Urinersatzlösung in einem Bereich auf den Inkontinenzartikel trifft, in welchem auch bei einem Miktionsereignis im Benutzungszustand die Miktionsflüssigkeit auf den Inkontinenzartikel treffen würde. Sollte dieser Bereich nicht zweifelsfrei feststehen, soll die Urinersatzlösung mittig auf den Absorptionskörper, insbesondere im Bereich einer Quermittelachse des Absorptionskörpers treffen. Falls der Inkontinenzartikel eine Flüssigkeitsaufnahmeschicht oder -verteilerschicht aufweist, wird die Urinersatzlösung insbesondere mittig innerhalb eines von der Flüssigkeitsaufnahmeschicht oder - verteilerschicht gebildeten Bereiches auf den Inkontinenzartikel gegossen.

Zwei Stunden nach Beginn der ersten simulierten Miktion mit dem ersten Volumen der Urinersatzlösung wird ein zweites Volumen der Urinersatzlösung, welches 20 % der maximalen Absorptionskapazität des Inkontinenzartikels entspricht, wie oben beschrieben auf den Inkontinenzartikel gegossen, um eine zweite Miktion zu simulieren.

Fünf Stunden nach Beginn der Beladung mit dem ersten Volumen der Urinersatzlösung wird ein drittes Volumen der Urinersatzlösung, welches 20 % der maximalen Absorptionskapazität des Inkontinenzartikels entspricht, wie oben beschrieben auf den Inkontinenzartikel gegossen, um eine dritte Miktion zu simulieren.

Nach jedem der drei simulierten Miktionsereignisse wird der pH-Wert an der äußeren Oberseite des Topsheets des Inkontinenzartikels 1, 3, 5, 10, 15 und 20 min nach Ende der jeweiligen simulierten Miktion gemessen. Dazu wird der pH-Wert an jeweils 4 Messpunkten gemessen, welche in Längs- und/oder Querrichtung des flach ausgebreiteten Inkontinenzartikels voneinander beabstandet sind und ein Durchschnittswert der vier gemessenen Werte gebildet. Falls eine Flüssigkeitsaufnahmeschicht oder -verteilerschicht vorhanden ist, sollen die Messungen innerhalb des von dieser Schicht gebildeten Bereiches erfolgen. In jedem Fall sind die Messpunkte innerhalb eines von pH-Regulationsmittel überfangenen Bereiches anzuordnen.

Die Messpunkte sollen voneinander beabstandet sein, insbesondere zumindest nicht miteinander überlappen. Es ist grundsätzlich darauf zu achten, dass die Messpunkte des Inkontinenzartikels nicht zu trocken sind, um verlässliche Messergebnisse zu erhalten. Bevorzugt sollen die Positionen der 4 Messpunkte bei allen Messungen beibehalten werden, jedoch ist auch möglich, die Position eines oder mehrerer Messpunkte zu variieren, beispielsweise falls ein Messpunkt zum Zeitpunkt einer späteren Messung zu trocken sein sollte. Das kann beispielsweise bei Inkontinenzartikeln mit besonders niedriger Rücknässung insbesondere 15 und/oder 20 Minuten nach der jeweiligen simulierten Miktion der Fall sein. Sollten nicht wenigstens zwei geeignete Messpunkte für eine Messung zur Verfügung stehen, wird die Messung nur zu den Zeitpunkten 1, 3, 5, 10 und gegebenenfalls 15 Minuten nach Ende der jeweiligen simulierten Miktion ausgewertet. Sollten für eine Messung nur zwei oder drei Messpunkte zur Verfügung stehen ist ein Durchschnittswert der zwei oder drei gemessenen Werte zu bilden.

Aufgrund des Routine-Charakters solcher pH-Messungen im Fachgebiet ist es dem Fachmann leicht möglich, eine Beurteilung vorzunehmen, ob ein möglicher Messpunkt zu trocken ist.

Die Messung kann grundsätzlich mit jedem Messgerät vorgenommen werden, das sich nach Einschätzung des Fachmanns für die Oberflächenmessung von pH-Werten eignet. Beispielsweise eignen sich die Elektroden der Modelle HI14142 oder HI1413 von HANNA Instruments. Das Messgerät wird vor Beginn der Messung entsprechend der Herstellerangaben kalibriert.

## Patentansprüche

1. Inkontinenzartikel für die Aufnahme von Körperausscheidungen, umfassend ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper, wobei der Inkontinenzartikel zwischen dem Topsheet und dem Backsheet zumindest bereichsweise ein pH-Regulationsmittel umfasst, wobei das pH-Regulationsmittel Mononatriumcitrat oder Dinatriumcitrat aufweist, wobei das pH-Regulationsmittel partikelförmig ist, wobei mindestens 50 Gewichtsprozent des pH-Regulationsmittels eine Partikelgröße von 10 bis 2000 µm aufweist.

2. Inkontinenzartikel nach Anspruch 1, wobei das pH-Regulationsmittel Trinatriumcitrat aufweist.

3. Inkontinenzartikel nach Anspruch 1, wobei das pH-Regulationsmittel aus Mononatriumcitrat besteht

4. Inkontinenzartikel nach Anspruch 1, wobei das pH-Regulationsmittel aus Dinatriumcitrat besteht.

5. Inkontinenzartikel nach einem oder mehreren der Ansprüche 1 bis 4, wobei mindestens 50 Gewichtsprozent des pH-Regulationsmittels eine Partikelgröße von 50 bis 1200 µm, insbesondere von 80 bis 800 µm aufweist.

6. Inkontinenzartikel nach einem oder mehreren der Ansprüche 1 bis 5, wobei die Menge an pH-Regulationsmittel 10 bis 100 g/m², insbesondere 20 bis 80 g/m², weiter insbesondere 25 bis 60 g/m² beträgt.

7. Inkontinenzartikel nach einem oder mehreren der Ansprüche 1 bis 6, wobei der Absorptionskörper mindestens eine Speicherschicht umfasst, die ein superabsorbierendes Polymer (SAP) aufweist.

8. Inkontinenzartikel nach Anspruch 7, wobei die Speicherschicht im Wesentlichen frei von dem pH-Regulationsmittel ist.

9. Inkontinenzartikel nach einem oder mehreren der Ansprüche 1 bis 8, wobei der Inkontinenzartikel eine Flüssigkeitsaufnahmeschicht umfasst, die eine dem Topsheet unmittelbar zugewandte Schicht des Absorptionskörpers bildet.

10. Inkontinenzartikel nach Anspruch 9, wobei das pH-Regulationsmittel im Wesentlichen zwischen der Flüssigkeitsaufnahmeschicht und einer dem Backsheet zugewandten Absorptionskörperschicht positioniert ist.

11. Inkontinenzartikel nach einem oder mehreren der Ansprüche 1 bis 10, wobei der Inkontinenzartikel über mindestens zwei, insbesondere mindestens drei Miktionsereignisse hinweg, einen hautfreundlichen pH-Wert aufrechterhält.

12. Inkontinenzartikel nach Anspruch 11, wobei der pH-Wert auf einer der Haut zugewandten Oberseite des Inkontinenzartikels einen Wert von 4.8-6.5, insbesondere 5.0-6.2, weiter insbesondere 5.2-6.0, weiter insbesondere 5.3-5.8, weiter insbesondere 5.4-5.6 beträgt.

## Claims

1. Incontinence article for absorption of bodily excretions, comprising an at least regionally liquid-permeable topsheet, a substantially liquid-impermeable backsheet, and an absorption body arranged between the topsheet and the backsheet, wherein the incontinence article between the topsheet and the backsheet comprises at least regionally a pH regulator, wherein the pH regulator comprises monosodium citrate or disodium citrate, wherein the pH regulator is particulate, wherein at least 50 percent by weight of the pH regulator has a particle size of 10 to 2000 µm.

2. Incontinence article according to Claim 1, wherein the pH regulator comprises trisodium citrate.

3. Incontinence article according to Claim 1, wherein the pH regulator consists of monosodium citrate.

4. Incontinence article according to Claim 1, wherein the pH regulator consists of disodium citrate.

5. Incontinence article according to one or more of Claims 1 to 4, wherein at least 50 percent by weight of the pH regulator has a particle size of 50 to 1200 pm, in particular of 80 to 800 µm.

6. Incontinence article according to one or more of Claims 1 to 5, wherein the amount of pH regulator is 10 to 100 g/m², in particular 20 to 80 g/m², further in particular 25 to 60 g/m².

7. Incontinence article according to one or more of Claims 1 to 6, wherein the absorption body comprises at least one storage layer which comprises a superabsorbent polymer (SAP).

8. Incontinence article according to Claim 7, wherein the storage layer is substantially free of the pH regulator.

9. Incontinence article according to one or more of Claims 1 to 8, wherein the incontinence article comprises a liquid-acquisition layer which forms a layer of the absorption body that directly faces the topsheet.

10. Incontinence article according to Claim 9, wherein the pH regulator is positioned substantially between the liquid-acquisition layer and an absorption body layer that faces the backsheet.

11. Incontinence article according to one or more of Claims 1 to 10, wherein the incontinence article maintains a skin-friendly pH over at least two, in particular at least three, micturition events.

12. Incontinence article according to Claim 11, wherein the pH on a top side of the incontinence article that faces the skin is a value of 4.8-6.5, in particular 5.0-6.2, further in particular 5.2-6.0, further in particular 5.3-5.8, further in particular 5.4-5.6.

## Revendications

1. Article d'incontinence pour le recueil d'excrétions corporelles, comprenant un voile intérieur perméable aux liquides au moins par zones, un voile extérieur pour l'essentiel imperméable aux liquides et un corps absorbant disposé entre le voile intérieur et le voile extérieur, l'article d'incontinence comprenant entre le voile intérieur et le voile extérieur, au moins par zones, un régulateur de pH, le régulateur de pH comprenant du citrate monosodique ou du citrate disodique, le régulateur de pH étant particulaire, au moins 50 pour cent en poids du régulateur de pH présentant une granulométrie de 10 à 2000 µm.

2. Article d'incontinence selon la revendication 1, dans lequel le régulateur de pH comprend du citrate trisodique.

3. Article d'incontinence selon la revendication 1, dans lequel le régulateur de pH est constitué de citrate monosodique.

4. Article d'incontinence selon la revendication 1, dans lequel le régulateur de pH est constitué de citrate disodique.

5. Article d'incontinence selon l'une ou plusieurs des revendications 1 à 4, dans lequel au moins 50 pour cent en poids du régulateur de pH a une granulométrie de 50 à 1200 pm, en particulier de 80 à 800 µm.

6. Article d'incontinence selon l'une ou plusieurs des revendications 1 à 5, dans lequel la quantité du régulateur de pH est de 10 à 100 g/m², en particulier de 20 à 80 g/m², plus particulièrement de 25 à 60 g/m².

7. Article d'incontinence selon l'une ou plusieurs des revendications 1 à 6, dans lequel le corps absorbant comprend au moins une couche de stockage, qui comprend un polymère superabsorbant (SAP).

8. Article d'incontinence selon la revendication 7, dans lequel la couche de stockage est pour l'essentiel exempte du régulateur de pH.

9. Article d'incontinence selon l'une ou plusieurs des revendications 1 à 8, l'article d'incontinence comprenant une couche réceptrice de liquide, qui forme une couche du corps absorbant immédiatement en regard du voile intérieur.

10. Article d'incontinence selon la revendication 9, dans lequel le régulateur de pH est pour l'essentiel positionné entre la couche réceptrice de liquide et une couche de corps absorbant en regard du voile extérieur.

11. Article d'incontinence selon l'une ou plusieurs des revendications 1 à 10, l'article d'incontinence maintenant un pH doux pour la peau sur au moins deux, en particulier au moins trois événements de miction.

12. Article d'incontinence selon la revendication 11, dans lequel le pH, sur une face supérieure dirigée vers la peau de l'article d'incontinence, a une valeur de 4,8-6,5, en particulier de 5,0-6,2, plus particulièrement de 5,2-6,0, plus particulièrement de 5,3-5,8, plus particulièrement de 5,4-5,6.
